(19) Europäisches Patentamt / European Patent Office / Office européen des brevets

(11) EP 4 424 780 A1

(12) EUROPEAN PATENT APPLICATION
published in accordance with Art. 153(4) EPC

(43) Date of publication:
04.09.2024 Bulletin 2024/36

(21) Application number: 22887187.7

(22) Date of filing: 28.10.2022

(51) International Patent Classification (IPC):
C08L 101/14 (2006.01)   A61F 13/15 (2006.01)
A61F 13/53 (2006.01)   A61L 15/18 (2006.01)
A61L 15/46 (2006.01)   B01J 20/26 (2006.01)
B01J 20/28 (2006.01)   C08F 8/14 (2006.01)
C08K 3/04 (2006.01)   C08L 33/02 (2006.01)

(52) Cooperative Patent Classification (CPC):
A61F 13/15; A61F 13/53; A61L 15/18; A61L 15/46;
B01J 20/26; B01J 20/28; C08F 8/14; C08K 3/04;
C08L 33/02; C08L 101/14

(86) International application number:
PCT/JP2022/040443

(87) International publication number:
WO 2023/074862 (04.05.2023 Gazette 2023/18)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.10.2021 JP 2021178145

(71) Applicant: Sumitomo Seika Chemicals Co., Ltd.
Kako-gun, Hyogo 675-0145 (JP)

(72) Inventor: MORISHIMA, Shota
Himeji-shi, Hyogo 672-8076 (JP)

(74) Representative: Mewburn Ellis LLP
Aurora Building
Counterslip
Bristol BS1 6BX (GB)

(54) **WATER-ABSORBING RESIN COMPOSITION, ABSORBENT, AND ABSORBENT ARTICLE**

(57) Provided is a water-absorbing resin composition comprising water-absorbing resin particles and activated carbon, the water-absorbing resin composition combining an excellent deodorizing effect with inhibition of blocking due to moisture absorption. The water-absorbing resin composition comprises water-absorbing resin particles and activated carbon having a median particle diameter of 100-600 μm and has a content of the activated carbon of 0.03-5 mass%.

FIG. 1

**Description**

TECHNICAL FIELD

The present invention relates to a water-absorbing resin composition, an absorber, and an absorbent article, and more particularly to a water-absorbing resin composition constituting an absorber suitably used in hygienic materials such as disposable diapers, sanitary napkins, and incontinence pads, and a method for producing the water-absorbing resin composition.

BACKGROUND ART

In recent years, water-absorbing resins have been widely used in the field of hygienic materials such as disposable diapers, sanitary napkins, and incontinence pads.

As such a water-absorbing resin, a crosslinked product of a polymer of a partially neutralized acrylic acid salt has excellent water absorbing capacity, and acrylic acid as a raw material thereof is easily industrially available, thus the crosslinked product of the polymer can be produced at a constant quality and at a low cost. Also, since the crosslinked product of the polymer has many advantages such as being less likely to cause decomposition or deterioration, it is considered to be a preferable water-absorbing resin.

An absorbent article such as a disposable diaper, a sanitary napkin, or an incontinence pad mainly includes an absorber that is disposed in a central portion and absorbs and holds body fluids excreted from a body such as urine and menstrual blood, a liquid-permeable surface sheet (top sheet) disposed on a side coming in contact with the body, and a liquid-impermeable back sheet (back sheet) disposed on a side opposite to the side coming in contact with the body. The absorber is usually composed of hydrophilic fibers such as pulp and a water-absorbing resin.

The water-absorbing resin particles used for producing such an absorber may cause blocking due to moisture absorption (phenomenon in which aggregation occurs between water-absorbing resin particles and flowability as a powder decreases). Such blocking due to moisture absorption may cause a decrease in production efficiency during production of the absorber and adversely affect the performance of the obtained absorber.

As a method for suppressing blocking due to such moisture absorption, for example, a method for producing water-absorbing resin particles using epoxy-based compounds as an internal-crosslinking agent and a surface-crosslinking agent is known (see Patent Document 1).

PRIOR ART DOCUMENT

PATENT DOCUMENT

Patent Document 1: Japanese Patent Laid-open Publication No. 2017-185485

SUMMARY OF THE INVENTION

PROBLEMS TO BE SOLVED BY THE INVENTION

As described above, as a method for suppressing blocking due to moisture absorption, for example, a method for producing water-absorbing resin particles using epoxy-based compounds as an internal-crosslinking agent and a surface-crosslinking agent is known.

Activated carbon is expected to have a hygroscopic effect in addition to the action of adsorbing organic substances and the like. Thus, the present inventors have attempted to combine water-absorbing resin particles and activated carbon to impart an effect of suppressing blocking due to moisture absorption.

However, as a result of examination by the present inventors, it has also been confirmed that blocking tends to be deteriorated when water-absorbing resin particles and activated carbon are combined and mixed simply. Therefore, it has been found that a water-absorbing resin composition in which specific water-absorbing resin particles and specific activated carbon are mixed at a specific content can solve the problem of blocking due to moisture absorption.

Under such circumstances, a main object of the present invention is to provide a water-absorbing resin composition containing water-absorbing resin particles and activated carbon, the water-absorbing resin composition having an excellent effect of suppressing blocking due to moisture absorption.

MEANS FOR SOLVING THE PROBLEM

The present inventor has conducted intensive studies in order to solve the above problems. As a result, the

2

present inventors have found that when water-absorbing resin particles and activated carbon having a median particle size of 100 μm or more and 600 μm or less are used, and the content of the activated carbon is set to a range of 0.03 mass% or more and 5 mass% or less, an excellent effect of suppressing blocking due to moisture absorption is exhibited unexpectedly. The present invention has been completed through further intensive studies with respect to such findings.

That is, the present invention provides an invention having the following configuration.

Item 1. A water-absorbing resin composition containing water-absorbing resin particles, and activated carbon having a median particle size of 100 μm or more and 600 μm or less,
in which a content of the activated carbon is 0.03 mass% or more and 5 mass% or less.
Item 2. The water-absorbing resin composition according to item 1, in which the activated carbon has a crushed shape.
Item 3. The water-absorbing resin composition according to item 1 or 2, in which the water-absorbing resin particles have a BET specific surface area of 0.01 m²/g or more and 0.20 m²/g or less.
Item 4. An absorber including the water-absorbing resin composition according to any one of items 1 to 3.
Item 5. An absorbent article including the absorber according to item 4.

ADVANTAGES OF THE INVENTION

The present invention can provide a water-absorbing resin composition containing water-absorbing resin particles and activated carbon, the water-absorbing resin composition having an excellent effect of suppressing blocking due to moisture absorption. Furthermore, the present invention can also provide an absorber and an absorbent article using the water-absorbing resin composition.

BRIEF DESCRIPTION OF THE DRAWINGS

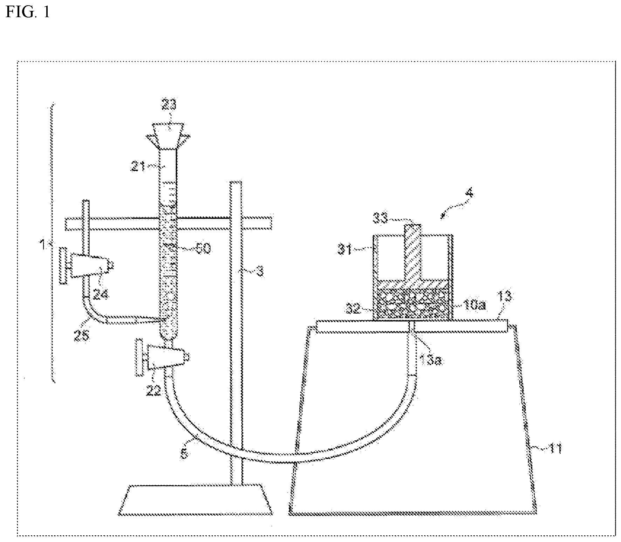

Fig. 1 is a schematic view of an apparatus for measuring a physiological saline absorption amount under a load of 4.14 kPa.

EMBODIMENTS OF THE INVENTION

In the present description, the term "comprising" includes "consisting essentially of" and "consisting of". In addition, in the present description, "(meth)acrylic" means" acrylic or methacrylic", and "(meth)acrylate" means" acrylate or methacrylate". The term "water-soluble" means that the solubility in water at 25°C is 5 mass% or more.

In addition, in the present description, a numerical value joined by "to" means a numerical range including numerical values before and after "to" as a lower limit value and an upper limit value. When a plurality of lower limit values and a plurality of upper limit values are described separately, any lower limit value and upper limit value can be selected and connected by "to".

1. Water-Absorbing Resin Composition

The water-absorbing resin composition of the present invention contains water-absorbing resin particles and activated carbon having a median particle size of 100 μm or more and 600 μm or less, and the content of the activated carbon is 0.03 mass% or more and 5 mass% or less. The water-absorbing resin composition of the present invention having such characteristics exhibits an excellent effect of suppressing blocking due to moisture absorption. Hereinafter, the water-absorbing resin composition of the present invention will be described in detail.

(Activated Carbon)

The activated carbon contained in the water-absorbing resin composition of the present invention has a median particle size of 100 μm or more and 600 μm or less. From the viewpoint of more suitably exerting the effect of the present invention, the median particle size of the activated carbon is preferably 200 μm or more, more preferably 300 μm or more, and preferably 550 μm or less, more preferably 500 μm or less, and examples of the preferable range include 200 to 550 μm and 300 to 500 μm. When the median particle size of the activated carbon is 100 μm or more, the contact area between the activated carbon and the water-absorbing resin particles is reduced, so that the aggregation of the water-absorbing resin particles due to the action of the activated carbon can be prevented to enhance the effect of suppressing blocking.

The median particle size (D 50 (median diameter), volume-based) of the activated carbon can be measured using a laser diffraction particle size distribution analyzer, and specifically, is a value measured by the method described in the examples.

From the viewpoint of more suitably exerting the effect of the present invention, the shape of the activated carbon is preferably a crushed shape, a columnar shape, or the like, and more preferably a crushed shape.

Further, from the viewpoint of more suitably exerting the effect of the present invention, the BET specific surface area of the activated carbon is preferably 100 $m^2$/g or more, more preferably 1000 $m^2$/g or more, and is preferably 3000 $m^2$/g or less, more preferably 2000 $m^2$/g or less, and examples of the preferable range include 100 to 3000 $m^2$/g and 1000 to 2000 $m^2$/g.

The BET specific surface area of activated carbon can be measured using a specific surface area analyzer, and specifically, is a value measured by the method described in the examples.

From the viewpoint of more suitably exerting the effect of the present invention, the activated carbon is preferably activated carbon having a polar functional group (hydrophilic functional group) on the surface (that is, hydrophilic activated carbon). Examples of the polar functional group include a hydroxy group, a carboxy group, and a phenol group. The activated carbon having a polar functional group on the surface is, for example, commercially available as activated carbon for a liquid phase, activated carbon for water treatment, and the like.

Examples of the origin of the activated carbon include coconut shells, infusible or carbonized organic materials, and infusible resins such as phenol resins. Examples of the organic material include polyacrylonitrile, pitch, polyvinyl alcohol, and cellulose. Among them, the origin of the activated carbon is preferably coconut shell or pitch (for example, coal pitch or cellulose pitch).

The content of activated carbon in the water-absorbing resin composition of the present invention is 0.03 mass% or more and 5 mass% or less. From the viewpoint of more suitably exerting the effect of the present invention, the content is preferably 0.05 mass% or more, more preferably 0.1 mass% or more, and is preferably 5 mass% or less, more preferably 3 mass% or less, still more preferably 1 mass% or less, and examples of the preferable range include 0.05 to 5 mass% and 0.1 to 3 mass%.

In the water-absorbing resin composition of the present invention, the activated carbon is preferably disposed on the surfaces of the water-absorbing resin particles (that is, the activated carbon is present on the surfaces of the water-absorbing resin particles). As described later, for example, by mixing the water-absorbing resin particles and the activated carbon in a solid phase state, the activated carbon adheres to the surfaces of the water-absorbing resin particles, and the activated carbon can be disposed on the surfaces of the water-absorbing resin particles.

From the viewpoint of more suitably exerting the effect of the present invention, the iodine adsorption amount of the activated carbon is preferably 100 mg/g or more, more preferably 500 mg/g or more, and is preferably 3000 mg/g or less, more preferably 2000 mg/g or less, and examples of the preferable range include 100 to 3000 mg/g and 500 to 2000 mg/g.

Here, the iodine adsorption amount of activated carbon is a value measured in accordance with JIS K1474.

From the viewpoint of more suitably exerting the effect of the present invention, the loss on drying of activated carbon is preferably 0.1% or more, more preferably 0.3% or more, and is preferably 10% or less, more preferably 5% or less, and examples of the preferable range include 0.1 to 10% and 0.3 to 5%.

Here, the loss on drying of activated carbon is a value measured in accordance with JIS K1474.

From the viewpoint of more suitably exerting the effect of the present invention, the pH of activated carbon is preferably 3 or more, more preferably 4 or more, or 5 or more, and is preferably 12 or less, more preferably 10 or less, and examples of the preferable range include 3 to 12, 4 to 10, and 5 to 10.

Here, the pH of activated carbon is a value measured in accordance with JIS K1474.

From the viewpoint of more suitably exerting the effect of the present invention, the residue on ignition of activated carbon is preferably 0.01 or more, more preferably 0.05 or more, and is preferably 7.0 or less, more preferably 2.0 or less, and examples of the preferable range include 0.01 to 7.0 and 0.05 to 2.0.

Here, the residue on ignition of activated carbon is a value measured in accordance with JIS K1474.

Next, the water-absorbing resin particles contained in the water-absorbing resin composition of the present invention will be described in detail.

(Water-Absorbing Resin Particles)

The water-absorbing resin particles contained in the water-absorbing resin composition of the present invention is composed of a crosslinked polymer obtained by crosslinking a polymer of a water-soluble ethylenically unsaturated monomer, that is, a crosslinked polymer having a structural unit derived from a water-soluble ethylenically unsaturated monomer.

From the viewpoint of more suitably exerting the effect of the present invention, the water absorption speed by the Vortex method of the water-absorbing resin particles is preferably 60 seconds or less, more preferably 50 seconds or less, and still more preferably 40 seconds or less, and is preferably 10 seconds or more, more preferably 15 seconds or more, and still more preferably 20 seconds or more, and examples of the preferable range include 10 to 60 seconds and 20 to 40 seconds.

The water absorption speed of the water-absorbing resin particles by the Vortex method is a value measured by the method described in the examples.

From the viewpoint of more suitably exerting the effect of the present invention, the physiological saline retention amount of the water-absorbing resin particles is preferably 20 g/g or more, more preferably 25 g/g or more, and still more preferably 30 g/g or more, and is preferably 60 g/g or less, more preferably 55 g/g or less, and still more preferably 50 g/g or less, and examples of the preferable range include 30 to 50 g/g.

In addition, from the viewpoint of more suitably exerting the effect of the present invention, the physiological saline absorption amount under a load of 4.14 kPa of the water-absorbing resin particles is preferably 10 ml/g or more, more preferably 13 ml/g or more, still more preferably 15 ml/g or more, and is preferably 40 ml/g or less, more preferably 35 ml/g or less, still more preferably 30 ml/g or less, and examples of the preferable range include 10 to 40 ml/g and 15 to 30 ml/g.

Each of the physiological saline retention amount and the physiological saline absorption amount under a load of 4.14 kPa of the water-absorbing resin particles is a value measured by the method described in the examples.

Further, from the viewpoint of more suitably exerting the effect of the present invention, the BET specific surface area of the water-absorbing resin particles is preferably 0.015 $m^2$/g or more, more preferably 0.02 $m^2$/g or more, and is preferably 0.180 $m^2$/g or less, more preferably 0.15 $m^2$/g or less, and examples of the preferable range include 0.01 to 0.180 $m^2$/g and 0.02 to 0.15 $m^2$/g.

The BET specific surface area of the water-absorbing resin particles can be measured using a specific surface area analyzer, and specifically, the BET specific surface area is a value measured by the method described in the examples.

In addition, from the viewpoint of more suitably exerting the effect of the present invention, the bulk density of the water-absorbing resin particles is preferably 0.3 g/mL or more, more preferably 0.6 g/mL or more, and is preferably 1 g/mL or less, more preferably 0.8 g/mL or less, and examples of the preferable range include 0.6 to 0.8 g/mL.

The bulk density of the water-absorbing resin particles can be measured using a "bulk specific gravity measuring apparatus" described in JIS-K-6720-2, and specifically, it is a value measured by a method described in the examples.

The water-absorbing resin is usually in particulate form. The median particle size of the water-absorbing resin particles is preferably 150 μm or more, 200 μm or more, 250 μm or more, 280 μm or more, 300 μm or more, or 350 μm or more from the viewpoint of suitably exerting the effect of the present invention while avoiding local absorption in the absorbent article. In addition, the median particle size is preferably 850 μm or less, 600 μm or less, 550 μm or less, 500 μm or less, 450 μm or less, or 400 μm or less from the viewpoint of suitably exerting the effect of the present invention while making the tactile sensation of the absorbent article comfortable. That is, the median particle size is preferably 150 to 850 μm, more preferably 200 to 600 μm, still more preferably 250 to 500 μm, even still more preferably 300 to 450 μm, and further preferably 320 to 400 μm. Also in the water-absorbing resin composition of the present invention, the median particle size is preferably 150 to 850 μm, more preferably 200 to 600 μm, still more preferably 250 to 500 μm, even still more preferably 300 to 450 μm, and further more preferably 320 to 400 μm.

Other than a form in which each of the water-absorbing resin particles is composed of a single particle, the water-absorbing resin particles may be in a form (secondary particles) in which fine particles (primary particles) are aggregated. Examples of the shape of the primary particle include a substantially spherical shape, an indefinite crushed shape, and a plate shape. Examples of the primary particles produced by reversed-phase suspension polymerization include substantially spherical single particles having a smooth surface shape such as a perfect spherical shape or an elliptical spherical shape.

The median particle size of the water-absorbing resin particles can be measured using JIS standard sieves, and specifically, is a value measured by the method described in the examples.

As a polymerization method of the water-soluble ethylenically unsaturated monomer, one of an aqueous solution polymerization method, an emulsion polymerization method, a reversed-phase suspension polymerization method, and the like, which are representative polymerization methods, is used. In the aqueous solution polymerization method, polymerization is performed by heating an aqueous solution of a water-soluble ethylenically unsaturated monomer while stirring the aqueous solution as necessary. In the reversed-phase suspension polymerization method, polymerization is performed by heating a water-soluble ethylenically unsaturated monomer in a hydrocarbon dispersion medium under stirring.

An example of the method for producing the water-absorbing resin particles will be described below.

Specific examples of the method for producing water-absorbing resin particles include a method for producing water-absorbing resin particles by performing reversed-phase suspension polymerization of a water-soluble ethylenically unsaturated monomer in a hydrocarbon dispersion medium, the method including a step of performing polymerization in the presence of a radical polymerization initiator and a step of surface-crosslinking a hydrous gel obtained by the polymerization in the presence of a surface-crosslinking agent. In the method for producing water-absorbing resin particles of the present invention, an internal-crosslinking agent may be added to the water-soluble ethylenically unsaturated monomer as necessary to form a hydrous gel having an internally crosslinked structure.

<Polymerization Step>

[Water-Soluble Ethylenically Unsaturated Monomer]

Examples of the water-soluble ethylenically unsaturated monomer include (meth)acrylic acid (in the present description, "acrylic" and "methacrylic" are collectively referred to as "(meth)acrylic", and the same applies hereinafter) and salts thereof; 2-(meth)acrylamide-2-methylpropanesulfonic acid and salts thereof; nonionic monomers such as (meth)acrylamide, N,N-dimethyl (meth)acrylamide, 2-hydroxyethyl (meth)acrylate, N-methylol (meth)acrylamide, and polyethylene glycol mono(meth)acrylate; amino group-containing unsaturated monomers such as N,N-diethylaminoethyl (meth)acrylate, N,N-diethylaminopropyl (meth)acrylate, and diethylaminopropyl (meth)acrylamide, and quaternized products thereof. Among these water-soluble ethylenically unsaturated monomers, from the viewpoints of industrial availability and the like, (meth)acrylic acid or salts thereof, (meth)acrylamide, and N,N-dimethylacrylamide are preferable, and (meth)acrylic acid and salts thereof are more preferable. These water-soluble ethylenically unsaturated monomers may be used singly or in combination of two or more.

Among them, acrylic acid and salts thereof are widely used as raw materials of the water-absorbing resin particles, and the acrylic acid and/or a salt thereof may be copolymerized with the above-described other water-soluble ethylenically unsaturated monomer and then used. In this case, the acrylic acid and/or a salt thereof is preferably used as a main water-soluble ethylenically unsaturated monomer in an amount of 70 to 100 mol% with respect to the total amount of water-soluble ethylenically unsaturated monomers.

The water-soluble ethylenically unsaturated monomer may be dispersed in a state of an aqueous solution in a hydrocarbon dispersion medium and subjected to reversed-phase suspension polymerization. When the water-soluble ethylenically unsaturated monomer is an aqueous solution, the dispersion efficiency in the hydrocarbon dispersion medium can be increased. The concentration of the water-soluble ethylenically unsaturated monomer in this aqueous solution is preferably in a range of 20 mass% to a saturated concentration or less. The concentration of the water-soluble ethylenically unsaturated monomer is more preferably 55 mass% or less, still more preferably 50 mass% or less, and even still more preferably 45 mass% or less. On the other hand, the concentration of the water-soluble ethylenically unsaturated monomer is more preferably 25 mass% or more, still more preferably 28 mass% or more, and even still more preferably 30 mass% or more.

Like (meth)acrylic acid, 2-(meth)acrylamide-2-methylpropanesulfonic acid, or similar monomer, when the water-soluble ethylenically unsaturated monomer has an acid group, the acid group may be neutralized with an alkaline neutralizing agent as necessary prior to use. Examples of such an alkaline neutralizing agent include alkali metal salts such as sodium hydroxide, sodium carbonate, sodium hydrogen carbonate, potassium hydroxide, and potassium carbonate; and ammonia. In addition, these alkaline neutralizing agents may be used in a form of an aqueous solution in order to simplify the neutralization operation. The alkaline neutralizing agents described above may be used singly or in combination of two or more.

The degree of neutralization of the water-soluble ethylenically unsaturated monomer by the alkaline neutralizing agent is preferably 10 to 100 mol%, more preferably 30 to 90 mol%, still more preferably 40 to 85 mol%, and even still more preferably 50 to 80 mol% as the degree of neutralization with respect to all the acid groups of the water-soluble ethylenically unsaturated monomer.

[Radical Polymerization Initiator]

Examples of the radical polymerization initiator added to the polymerization step include persulfates such as potassium persulfate, ammonium persulfate, and sodium persulfate, peroxides such as methyl ethyl ketone peroxide, methyl isobutyl ketone peroxide, di-t-butyl peroxide, t-butyl cumyl peroxide, t-butyl peroxyacetate, t-butyl peroxyisobutyrate, t-butyl peroxypivalate, and hydrogen peroxide, and azo compounds such as 2,2'-azobis(2-amidinopropane) dihydrochloride, 2,2'-azobis[2-(N-phenylamidino)propane] dihydrochloride, 2,2'-azobis[2-(N-allylamidino)propane] dihydrochloride, 2,2'-azobis{2-[1-(2-hydroxyethyl)-2-imidazolin-2-yl]propane} dihydrochloride, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). Among these radical polymerization initiators, potassium persulfate, ammonium persulfate, sodium persulfate, and 2,2'-azobis(2-amidinopropane) dihydrochloride are preferable from the viewpoints of easy availability and easy handling. These radical polymerization initiators may be used singly or in combination of two or more. The radical polymerization initiator can also be used as a redox polymerization initiator in combination with a reducing agent such as sodium sulfite, sodium hydrogen sulfite, ferrous sulfate, or L-ascorbic acid.

The amount of the radical polymerization initiator used is, for example, 0.00005 to 0.01 mol with respect to 1 mol of the water-soluble ethylenically unsaturated monomer. By satisfying such an amount used, occurrence of a rapid polymerization reaction can be avoided, and the polymerization reaction can be completed in an appropriate time.

[Internal-Crosslinking Agent]

Examples of the internal-crosslinking agent include those capable of crosslinking a polymer of a water-soluble ethylenically unsaturated monomer to be used, and examples thereof include (poly)ethylene glycol ["(poly)" means the case where there is or is not a prefix "poly", and the same applies hereinafter], unsaturated polyesters obtained by reacting polyols including diols and triols such as (poly)propylene glycol, 1,4-butanediol, 1,6-hexanediol, trimethylolpropane, and (poly)glycerin with unsaturated acids such as (meth)acrylic acid, maleic acid, and fumaric acid; bisacrylamides such as N,N-methylenebisacrylamide; di(meth)acrylic acid esters or tri(meth)acrylic acid esters obtained by reacting polyepoxide with (meth)acrylic acid; di(meth)acrylic acid carbamyl esters obtained by reacting polyisocyanates such as tolylene diisocyanate and hexamethylene diisocyanate with hydroxyethyl (meth)acrylate; compounds having two or more polymerizable unsaturated groups, such as allylated starch, allylated cellulose, diallyl phthalate, N,N',N"-triallyl isocyanurate, and divinylbenzene; polyglycidyl compounds including diglycidyl compounds such as (poly)ethylene glycol diglycidyl ether, (poly)propylene glycol diglycidyl ether, and (poly)glycerin diglycidyl ether and triglycidyl compounds; epihalohydrin compounds such as epichlorhydrin, epibromhydrin, and $\alpha$-methylepichlorhydrin; compounds having two or more reactive functional groups including isocyanate compounds such as 2,4-tolylene diisocyanate and hexamethylene diisocyanate; and oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol. Among these internal-crosslinking agents, polyglycidyl compounds are preferably used, diglycidyl ether compounds are more preferably used, and (poly)ethylene glycol diglycidyl ether, (poly)propylene glycol diglycidyl ether, and (poly)glycerin diglycidyl ether are preferably used. These internal-crosslinking agents may be used singly or in combination of two or more.
The amount of the internal-crosslinking agent used is preferably 0.000001 to 0.02 mol, more preferably 0.00001 to 0.01 mol, still more preferably 0.00001 to 0.005 mol, and even still more preferably 0.00005 to 0.002 mol with respect to 1 mol of the water-soluble ethylenically unsaturated monomer.

[Hydrocarbon Dispersion Medium]

Examples of the hydrocarbon dispersion medium include aliphatic hydrocarbons having 6 to 8 carbon atoms such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. Among these hydrocarbon dispersion media, n-hexane, n-heptane, and cyclohexane are particularly suitably used because they are industrially easily available, have stable quality, and are inexpensive. These hydrocarbon dispersion media may be used singly or in combination of two or more. As an example of the mixture of the hydrocarbon dispersion media, a suitable result can be obtained also by using a commercially available product such as Exxsol Heptane (manufactured by Exxon Mobil Corporation: containing 75 to 85 mass% hydrocarbons of heptane and an isomer thereof).
The amount of the hydrocarbon dispersion medium used is preferably 100 to 1500 parts by mass, and more preferably 200 to 1400 parts by mass with respect to 100 parts by mass of the first-stage water-soluble ethylenically unsaturated monomer from the viewpoints of uniformly dispersing the water-soluble ethylenically unsaturated monomer and easily controlling the polymerization temperature. As will be described later, the reversed-phase suspension polymerization is performed in one stage (single stage) or two or more stages, and the above-described first-stage polymerization means the first-stage polymerization reaction in the single-stage polymerization or the multistage polymerization (the same applies hereinafter).

[Dispersion Stabilizer]

(Surfactant)

In the reversed-phase suspension polymerization, a dispersion stabilizer can also be used in order to improve the dispersion stability of the water-soluble ethylenically unsaturated monomer in the hydrocarbon dispersion medium. As the dispersion stabilizer, a surfactant can be used.
As the surfactant, for example, a sucrose fatty acid ester, a polyglycerin fatty acid ester, a sorbitan fatty acid ester, a polyoxyethylene sorbitan fatty acid ester, a polyoxyethylene glycerin fatty acid ester, a sorbitol fatty acid ester, a polyoxyethylene sorbitol fatty acid ester, a polyoxyethylene alkyl ether, a polyoxyethylene alkyl phenyl ether, a polyoxyethylene castor oil, a polyoxyethylene hydrogenated castor oil, an alkylallyl formaldehyde condensed polyoxyethylene ether, a polyoxyethylene polyoxypropylene block copolymer, a polyoxyethylene polyoxypropyl alkyl ether, a polyethylene glycol fatty acid ester, an alkyl glucoside, an N-alkyl gluconamide, a polyoxyethylene fatty acid amide, a polyoxyethylene alkylamine, a phosphoric acid ester of a polyoxyethylene alkyl ether, a phosphoric acid ester of a polyoxyethylene alkyl allyl ether, or the like can be used. Among these surfactants, it is particularly preferable to use a sorbitan fatty acid ester, a polyglycerin fatty acid ester, or a sucrose fatty acid ester from the viewpoint of dispersion stability of the monomer. These surfactants may be used singly or in combination of two or more.

The amount of the surfactant used is preferably 0.1 to 30 parts by mass, and more preferably 0.3 to 20 parts by mass with respect to 100 parts by mass of the first-stage water-soluble ethylenically unsaturated monomer.

(Polymeric Dispersant)

As the dispersion stabilizer used in the reversed-phase suspension polymerization, a polymeric dispersant may be used together with the surfactant described above.

Examples of the polymeric dispersant include maleic anhydride-modified polyethylene, maleic anhydride-modified polypropylene, maleic anhydride-modified ethylene-propylene copolymer, maleic anhydride-modified ethylene-propylene-diene terpolymer (EPDM), maleic anhydride-modified polybutadiene, maleic anhydride-ethylene copolymer, maleic anhydride-propylene copolymer, maleic anhydride-ethylene-propylene copolymer, maleic anhydride-butadiene copolymer, polyethylene, polypropylene, ethylene-propylene copolymer, oxidized polyethylene, oxidized polypropylene, oxidized ethylene-propylene copolymer, ethylene-acrylic acid copolymer, ethyl cellulose, and ethyl hydroxyethyl cellulose. Among these polymeric dispersants, maleic anhydride-modified polyethylene, maleic anhydride-modified polypropylene, maleic anhydride-modified ethylene-propylene copolymer, maleic anhydride-ethylene copolymer, maleic anhydride-propylene copolymer, maleic anhydride-ethylene-propylene copolymer, polyethylene, polypropylene, ethylene-propylene copolymer, oxidized polyethylene, oxidized polypropylene, and oxidized ethylene-propylene copolymer are particularly preferably used from the viewpoint of dispersion stability of monomers. These polymeric dispersants may be used singly or in combination of two or more.

The amount of the polymeric dispersant used is preferably 0.1 to 30 parts by mass, and more preferably 0.3 to 20 parts by mass with respect to 100 parts by mass of the first-stage water-soluble ethylenically unsaturated monomer.

[Other Components]

In the method for producing water-absorbing resin particles, if desired, other components may be added to an aqueous solution containing the water-soluble ethylenically unsaturated monomer, and then the reversed-phase suspension polymerization may be performed. As other components, various additives such as a thickener and a chain transfer agent can be added.

As an example, reversed-phase suspension polymerization can be performed after adding a thickener to an aqueous solution containing the water-soluble ethylenically unsaturated monomer. By thus adding a thickener to adjust the viscosity of the aqueous solution, it is possible to control the median particle size obtained in the reversed-phase suspension polymerization.

As the thickener, for example, hydroxyethyl cellulose, hydroxypropyl cellulose, methyl cellulose, carboxymethyl cellulose, polyacrylic acid, a (partial) neutralized polyacrylic acid, polyethylene glycol, polyacrylamide, polyethyleneimine, dextrin, sodium alginate, polyvinyl alcohol, polyvinylpyrrolidone, polyethylene oxide, or the like can be used. When the stirring speed during polymerization is the same, the primary particles and/or the secondary particles of the obtained particles tend to be larger as the viscosity of the aqueous solution of the water-soluble ethylenically unsaturated monomer is higher.

[Reversed-Phase Suspension Polymerization]

In performing the reversed-phase suspension polymerization, for example, an aqueous monomer solution containing a water-soluble ethylenically unsaturated monomer is dispersed in a hydrocarbon dispersion medium in the presence of a dispersion stabilizer. In this case, as long as it is before start of the polymerization reaction, the addition timing of the dispersion stabilizer (surfactant or polymeric dispersant) may be either before or after the addition of the aqueous monomer solution.

Among them, from the viewpoint of easily reducing the amount of the hydrocarbon dispersion medium remaining in the resulting water-absorbing resin particles, it is preferable to disperse the aqueous monomer solution in the hydrocarbon dispersion medium in which the polymeric dispersant has been dispersed, and then to further disperse the surfactant therein before performing polymerization.

Such reversed-phase suspension polymerization can be performed in one stage or two or more stages. In addition, from the viewpoint of enhancing productivity, it is preferable to perform 2 to 3 stages.

When the reversed-phase suspension polymerization is performed in two or more stages, the first-stage reversed-phase suspension polymerization is performed, then the water-soluble ethylenically unsaturated monomer is added to and mixed with the reaction mixture obtained in the first-stage polymerization reaction, and the second-stage or subsequent reversed-phase suspension polymerization may be performed in the same manner as the first-stage polymerization. In the reversed-phase suspension polymerization in each of the second and subsequent stages, in addition to the water-soluble ethylenically unsaturated monomer, a radical polymerization initiator is preferably added within the range of the molar ratio of each component to the water-soluble ethylenically unsaturated monomer described above based on the amount of the water-soluble ethylenically unsaturated monomer added in the reversed-phase suspension polymerization in each of the second and subsequent stages, to perform reversed-phase suspension polymerization. In the second and subsequent polymerization, an internal-crosslinking agent may be added to the water-soluble ethylenically unsaturated monomer as necessary.

The reaction temperature of the polymerization reaction is preferably 20 to 110°C and more preferably 40 to 90°C from the viewpoints of enhancing the economic efficiency by allowing the polymerization to proceed rapidly and shortening the polymerization time, and allowing the reaction to proceed smoothly by easily removing the heat of polymerization.

<Surface-Crosslinking Step>

Next, the water-absorbing resin particles of the present invention are obtained by adding a surface-crosslinking agent to the hydrous gel having an internally crosslinked structure that has been obtained by polymerizing the water-soluble ethylenically unsaturated monomer, and crosslinking the hydrous gel (surface-crosslinking reaction). This surface-crosslinking reaction is preferably performed in the presence of a surface-crosslinking agent after polymerization of the water-soluble ethylenically unsaturated monomer. As described above, by subjecting the hydrous gel having an internally crosslinked structure to a surface-crosslinking reaction after polymerization, it is possible to obtain water-absorbing resin particles in which the crosslinking density in the vicinity of the surface of the water-absorbing resin particle is increased and various performances such as water absorption capacity under load are improved.

Examples of the surface-crosslinking agent include compounds having two or more reactive functional groups. Examples include polyols such as ethylene glycol, propylene glycol, 1,4-butanediol, diethylene glycol, triethylene glycol, trimethylolpropane, glycerin, polyoxyethylene glycol, polyoxypropylene glycol, and polyglycerin; polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether; haloepoxy compounds such as epichlorhydrin, epibromhydrin, and $\alpha$-methylepichlorhydrin; isocyanate compounds such as 2,4-tolylene diisocyanate and hexamethylene diisocyanate; oxetane compounds such as 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, and 3-butyl-3-oxetaneethanol; oxazoline compounds such as 1,2-ethylene bisoxazoline; carbonate compounds (for example, alkylene carbonate) such as ethylene carbonate, propylene carbonate, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxolan-2-one; and hydroxyalkylamide compounds such as bis[N,N-di($\beta$-hydroxyethyl)]adipamide. Among these surface-crosslinking agents, polyglycidyl compounds such as (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether are preferable. These surface-crosslinking agents may be used singly or in combination of two or more.

The amount of the surface-crosslinking agent used is preferably 0.00001 to 0.01 mol, more preferably 0.00005 to 0.005 mol, and still more preferably 0.0001 to 0.002 mol with respect to 1 mol of the total amount of the water-soluble ethylenically unsaturated monomers used for polymerization.

As a method for adding the surface-crosslinking agent, the surface-crosslinking agent may be added as it is or as an aqueous solution, or may be added as a solution using a hydrophilic organic solvent as a solvent as necessary. Examples of the hydrophilic organic solvent include lower alcohols such as methyl alcohol, ethyl alcohol, n-propyl alcohol, and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide. These hydrophilic organic solvents may be used singly or in combination of two or more, or as a mixed solvent obtained by mixing with water.

The surface-crosslinking agent may be added at any timing as long as it is added after almost all the polymerization reaction of the water-soluble ethylenically unsaturated monomer is completed. The surface-crosslinking agent is preferably added in the presence of moisture in a range of 1 to 400 parts by mass, more preferably in the presence of moisture in a range of 5 to 200 parts by mass, still more preferably in the presence of moisture in a range of 10 to 100 parts by mass, and even still more preferably in the presence of moisture in a range of 20 to 60 parts by mass with respect to 100 parts by mass of the water-soluble ethylenically unsaturated monomer. The amount of moisture means the total amount of moisture contained in the reaction system and moisture used as necessary when the surface-crosslinking agent is added.

The reaction temperature in the surface-crosslinking reaction is preferably 50 to 250°C, more preferably 60 to 180°C, still more preferably 60 to 140°C, and even still more preferably 70 to 120°C. The reaction time of the surface-crosslinking reaction is preferably 1 to 300 minutes, and more preferably 5 to 200 minutes.

<Drying Step>

The method may include a drying step of removing water, a hydrocarbon dispersion medium, and the like by externally applying energy such as heat and carrying out distillation after performing the reversed-phase suspension polymerization described above. When dehydration of the hydrous gel after the reversed-phase suspension polymerization is performed, the system in which the hydrous gel is dispersed in the hydrocarbon dispersion medium is heated, so that water and the hydrocarbon dispersion medium are temporarily distilled off to the outside of the system by azeotropic distillation. At this time, when only the distilled hydrocarbon dispersion medium is returned into the system, continuous azeotropic distillation becomes possible. Since the temperature in the system during drying is maintained at an azeotropic temperature with the hydrocarbon dispersion medium or lower in that case, it is preferable from the viewpoint that the resin is less likely to deteriorate. Subsequently, water and the hydrocarbon dispersion medium are distilled off to obtain the water-absorbing resin particles. Various performances of the water-absorbing resin particles to be obtained can be controlled by controlling the treatment conditions in the drying step after the polymerization to adjust the amount of water to be removed.

In the drying step, the drying treatment by distillation may be performed under normal pressure or under reduced pressure. From the viewpoint of enhancing the drying efficiency, the drying may be performed under a flow of nitrogen or the like. When the drying treatment is performed under normal pressure, the drying temperature is preferably 70 to 250°C, more preferably 80 to 180°C, still more preferably 80 to 140°C, and even still more preferably 90 to 130°C. When the drying treatment is performed under reduced pressure, the drying temperature is preferably 40 to 160°C, and more preferably 50 to 110°C.

When the surface-crosslinking step with the surface-crosslinking agent is performed after the polymerization of the monomer by reversed-phase suspension polymerization, the drying step by distillation described above is performed after the completion of the surface-crosslinking step. Alternatively, the surface-crosslinking step and the drying step may be performed simultaneously.

The water-absorbing resin composition of the present invention may contain an additive according to the purpose. Examples of such an additive include inorganic powders, surfactants, oxidizing agents, reducing agents, metal chelating agents, radical chain inhibitors, antioxidants, and antibacterial agents. For example, the flowability of the water-absorbing resin composition can be further improved by adding 0.05 to 5 parts by mass of amorphous silica as an inorganic powder with respect to 100 parts by mass of the water-absorbing resin particles. The additive is preferably hydrophilic or water-soluble.

In the water-absorbing resin composition of the present invention, the content of the water-absorbing resin particles (excluding additives) is preferably 70 mass% or more, more preferably 80 mass% or more, and still more preferably 90 mass% or more.

The water-absorbing resin composition of the present invention can be produced, for example, by mixing the water-absorbing resin particles and the activated carbon in a solid phase state.

2. Absorber and Absorbent Article

The water-absorbing resin composition of the present invention constitutes an absorber used in, for example, hygienic materials such as sanitary products and disposable diapers, and is suitably used in an absorbent article including the absorber.

Here, the absorber using the water-absorbing resin composition of the present invention contains the particulate water-absorbing resin composition of the present invention. The absorber may further contain hydrophilic fibers. Examples of the configuration of the absorber include a sheet-like structure in which water-absorbing resin particles are fixed on a nonwoven fabric or between a plurality of nonwoven fabrics, a mixed dispersion obtained by mixing a particulate water-absorbing resin composition and hydrophilic fibers so as to have a uniform composition, a sandwich structure in which a particulate water-absorbing resin composition is sandwiched between layered hydrophilic fibers, and a structure in which a particulate water-absorbing resin composition and hydrophilic fibers are wrapped with tissue. The absorber may contain other components, for example, an adhesive binder such as a heat-fusible synthetic fiber, a hot melt adhesive, or an adhesive emulsion for enhancing the shape retention of the absorber.

The content of the water-absorbing resin composition in the absorber is preferably 5 to 100 mass%, more preferably 10 to 95 mass%, still more preferably 20 to 90 mass%, and even still more preferably 30 to 80 mass%.

Examples of the hydrophilic fibers include cellulose fibers obtained from wood such as fluff pulp, mechanical pulp, chemical pulp, and semi-chemical pulp, artificial cellulose fibers such as rayon and acetate, and hydrophilized fibers made of synthetic resins such as polyamide, polyester, and polyolefin. The average fiber length of the hydrophilic fibers is usually 0.1 to 10 mm or may be 0.5 to 5 mm.

The absorber using the particulate water-absorbing resin composition of the present invention can be held between a liquid-permeable sheet (top sheet) through which a liquid can pass and a liquid-impermeable sheet (back sheet) through which a liquid cannot pass to form the absorbent article of the present invention. The liquid-permeable sheet is disposed on the side coming in contact with a body, and the liquid-impermeable sheet is disposed on a side opposite to the side coming in contact with the body.

Examples of the liquid-permeable sheet include an air-through type, a spunbond type, a chemical bond type, a needle punch type, and similar type nonwoven fabrics made of fibers such as polyethylene, polypropylene, and polyester, and porous synthetic resin sheets. Examples of the liquid-impermeable sheet include synthetic resin films made of resins such as polyethylene, polypropylene, and polyvinyl chloride.

EXAMPLES

Hereinafter, the present invention will be described in detail with reference to the examples and the comparative examples. However, the present invention is not limited to the examples.

The water-absorbing resin particles obtained in the following production example and the water-absorbing resin compositions obtained in the examples and the comparative examples were evaluated in the following various tests. Unless otherwise specified, the measurement was performed under an environment of a temperature of $25 \pm 2°C$ and a humidity of $50 \pm 10\%$.

[Production of Water-Absorbing Resin Particles]

<Production Example 1>

A 2-L round-bottom cylindrical separable flask having an inner diameter of 11 cm and equipped with a reflux condenser, a dropping funnel, a nitrogen gas inlet tube, and a stirring blade having two stages of 4-inclined paddle blades having a blade diameter of 5 cm as a stirrer was prepared. In this flask, 293 g of n-heptane as a hydrocarbon dispersion medium was placed, 0.736 g of a maleic anhydride-modified ethylene-propylene copolymer (Mitsui Chemicals, Inc., Hi-WAX 1105A) as a polymeric dispersant was added thereto, the temperature was raised to 80°C with stirring to dissolve the dispersant, and then the contents were cooled to 50°C. In a beaker having an internal volume of 300 mL, 92.0 g (1.03 mol) of an 80.5 mass% aqueous acrylic acid solution as a water-soluble ethylenically unsaturated monomer was placed, 147.7 g of a 20.9 mass% aqueous sodium hydroxide solution was added dropwise while cooling with ice water to perform neutralization of 75 mol%, and then 0.092 g of hydroxyethyl cellulose (Sumitomo Seika Chemicals Co., Ltd., HEC AW-15F) as a thickener, 0.0736 g (0.272 mmol) of potassium persulfate as a water-soluble radical polymerization agent, and 0.010 g (0.057 mmol) of ethylene glycol diglycidyl ether as an internal-crosslinking agent were added thereto and dissolved, thereby preparing a first-stage aqueous solution. Then, the aqueous solution prepared above was added to the separable flask and stirred for 10 minutes, after which a surfactant solution obtained by heating and dissolving 0.736 g of a sucrose stearate having an HLB of 3 (Mitsubishi Chemical Foods Corporation, RYOTO Sugar Ester S-370) as a surfactant in 6.62 g of n-heptane in a 20 mL-vial was further added. While stirring at a stirrer rotational speed of 550 rpm, the inside of the system was sufficiently purged with nitrogen, and then the flask was heated by immersing in a water bath at 70°C, and polymerization was performed for 60 minutes to obtain a first-stage polymerization slurry solution.

In another beaker having an internal volume of 500 mL, 128.8 g (1.43 mol) of an 80.5 mass% aqueous acrylic acid solution as a water-soluble ethylenically unsaturated monomer was placed, 159.0 g of a 27 mass% aqueous sodium hydroxide solution was added dropwise while cooling with ice water to perform neutralization of 75 mol%, and then 0.103 g (0.381 mmol) of potassium persulfate as a water-soluble radical polymerization initiator and 0.0117 g (0.067 mmol) of ethylene glycol diglycidyl ether as an internal-crosslinking agent were added thereto and dissolved, thereby preparing a second-stage aqueous solution.

While stirring at a stirrer rotational speed of 1000 rpm, the inside of the separable flask system was cooled to 25°C. Then the whole amount of the second-stage aqueous solution was added to the first-stage polymerization slurry. After the inside of the system was purged with nitrogen for 30 minutes, the flask was heated by immersing in a water bath at 70°C again, and polymerization was performed for 60 minutes to obtain a hydrous gel polymer.

Thereafter, the flask was immersed in an oil bath set at 125°C, and 256.2 g of water was removed to the outside of the system while refluxing n-heptane by azeotropic distillation of n-heptane and water. Then, 4.42 g of a 2 mass% aqueous solution of ethylene glycol diglycidyl ether (0.507 mmol) as a surface-crosslinking agent was added to the flask, and the contents were held at 83°C for 2 hours.

Thereafter, n-heptane was evaporated at 125°C, dried, and further passed through a sieve with an aperture of 850 $\mu$m to obtain 226.4 g of water-absorbing resin particles. The physiological saline retention amount of the water-absorbing resin particles was 39 g/g, the water absorption speed thereof was 35 seconds, the median particle size

thereof was 348 $\mu$m, the physiological saline absorption amount under a load of 4.14 kPa thereof was 22 ml/g, the bulk density thereof was 0.70 g/mL, and the BET specific surface area thereof was 0.032 m$^2$/g.

[Evaluation of Water-Absorbing Resin Particles]

<Physiological Saline Retention Amount>

The water-absorbing resin particles, 2.0 g, were weighed out in a cotton bag (cotton broadcloth No. 60, width 100 mm $\times$ length 200 mm), which was placed in a 500 ml beaker. A 0.9 mass% sodium chloride aqueous solution (physiological saline), 500 g, was poured into the cotton bag containing the water-absorbing resin particles at a time so as not to form lumps, the upper portion of the cotton bag was tied with a rubber band, and the cotton bag was allowed to stand for 30 minutes to swell the water-absorbing resin particles. The cotton bag after 30 minutes was dehydrated for 1 minute using a dehydrator (product number: H-122, manufactured by KOKUSAN Co., Ltd.) set so as to have a centrifugal force of 167 G, and the mass of the cotton bag containing the swollen gel after dehydration Wd (g) was measured. The same operation was performed without adding the water-absorbing resin particles, the empty mass of the cotton bag in a wet state We (g) was measured, and the physiological saline retention amount was calculated from the following formula.

$$\text{Physiological saline retention amount (g/g)} = [Wd - We]/2.0$$

<Measurement of Water Absorption Speed by Vortex Method>

In a 100 ml beaker, 50 $\pm$ 0.1 g of physiological saline adjusted to a temperature of 25 $\pm$ 0.2°C in a thermostatic water bath was weighed out, and stirred with a magnetic stirrer bar (8 mm$\varphi$ $\times$ 30 mm, no ring) to generate a vortex at a rotational speed of 600 rpm. The water-absorbing resin particles, 2.0 $\pm$ 0.002 g, were added to the physiological saline at a time, the time (second) from the addition of the water-absorbing resin particles to the time point at which the vortex on the liquid surface converged was measured, and the time was taken as the water absorption speed of the water-absorbing resin particles. This water absorption speed is also expressed as Vortex method or vortex time.

<Median Particle Size (Particle Size Distribution)>

Water-absorbing resin particles, 50 g, were used for measuring the median particle size (particle size distribution). JIS standard sieves were combined in the following order from the top: a sieve with an aperture of 850 $\mu$m, a sieve with an aperture of 500 $\mu$m, a sieve with an aperture of 425 $\mu$m, a sieve with an aperture of 300 $\mu$m, a sieve with an aperture of 250 $\mu$m, a sieve with an aperture of 180 $\mu$m, a sieve with an aperture of 150 $\mu$m, and a receptacle. The water-absorbing resin particles were put into the uppermost sieve of the combined sieves, and shaken for 20 minutes using a rotating and tapping shaker to perform classification. After classification, the mass of the water-absorbing resin particles retained on each sieve was calculated as a mass percentage with respect to the total amount, and the particle size distribution was determined. With respect to this particle size distribution, by calculating cumulative mass percentages of the retained on the sieves in descending order of particle size, the relationship between the aperture of the sieve and the cumulative value of the mass percentage of the water-absorbing resin particles retained on the sieve was plotted on a logarithmic probability paper. The plotted points on the probability paper were connected with a straight line, and a particle size corresponding to a cumulative mass percentage of 50 mass% was defined as a median particle size.

<Bulk Density>

The bulk density of the water-absorbing resin particles was measured using a "bulk specific gravity measuring apparatus" described in JIS-K-6720-2. About 120 mL of the water-absorbing resin particles were placed in a funnel part of the apparatus with the bottom closed by a damper, and the damper of the apparatus with a 100 mL volume receiver (inner diameter: 40 mmcp, cylindrical shape) installed 38 mm below the damper was quickly pulled out to drop the water-absorbing resin particles into the receiver. The water-absorbing resin particles raised from the receiver were scraped off with a flat plate, and then the mass W1 was measured together with the receiver. Separately, a value S (g/mL) was determined by subtracting the measured mass of the receiver in the empty state WO from the mass W 1 to obtain the mass of the water-absorbing resin particles and dividing the mass of the water-absorbing resin particles by the volume VmL of the receiver (the receiver V = 100 mL).

$$S \text{ (g/mL)} = [W1 - W0] \text{ (g)}/V \text{ (mL)}$$

In the same manner as described above, S was measured three times in total, and the average value thereof was taken as the bulk density.

<Physiological Saline Absorption Amount under a Load of 4.14 kPa>

The physiological saline absorption amount under a load of 4.14 kPa (water absorption amount under load) was measured using a measuring apparatus schematically shown in Fig. 1. The measurement was performed twice for one kind of water-absorbing resin particles, and the average value was obtained. The measuring apparatus includes a burette portion 1, a clamp 3, a conduit 5, a rack 11, a measuring table 13, and a measuring unit 4 placed on the measuring table 13. The burette portion 1 includes a burette tube 21 with printed scale, a rubber stopper 23 for hermetically sealing an upper opening of the burette tube 21, a cock 22 connected to a tip of a lower portion of the burette tube 21, and an air introduction tube 25 connected to a lower portion of the burette tube 21, and a cock 24. The burette portion 1 is fixed by the clamp 3. The flat plate-shaped measuring table 13 has a through hole 13a having a diameter of 2 mm formed in a central portion thereof, and is supported by the rack 11 that is height adjustable. The through hole 13a of the measuring table 13 and the cock 22 of the burette portion 1 are connected by the conduit 5. The inner diameter of the conduit 5 is 6 mm.

The measuring unit 4 includes a cylinder 31 made of PLEXIGLAS, a polyamide mesh 32 bonded to one opening of the cylinder 31, and a weight 33 movable in the vertical direction in the cylinder 31. The cylinder 31 is placed on the measuring table 13 with the polyamide mesh 32 interposed therebetween. The inner diameter of the cylinder 31 is 20 mm. The aperture of the polyamide mesh 32 is 75 $\mu$m (200 mesh). The weight 33 has a diameter of 19 mm and a mass of 119.6 g, and a load of 4.14 kPa (0.6 psi) can be applied to the water-absorbing resin particles 10a uniformly disposed on the polyamide mesh 32, as described later.

First, the cock 22 and the cock 24 of the burette portion 1 were closed, and 0.9 mass% physiological saline adjusted to 25°C was put into the burette tube 21 through the opening at the upper portion of the burette tube 21. Next, the upper opening of the burette tube 21 was sealed with the rubber stopper 23, and then the cock 22 and the cock 24 were opened. The inside of the conduit 5 was filled with 0.9 mass% saline 50 such that air bubbles did not enter. The height of the measuring table 13 was adjusted such that the height of the water surface of the 0.9 mass% saline reaching the inside of the through hole 13a was the same as the height of the upper surface of the measuring table 13. After the adjustment, the height of the water surface of the 0.9 mass% saline 50 in the burette tube 21 was determined by reading the scale of the burette tube 21, and the position was defined as a 0 point (a read at 0 second).

In the measuring unit 4, 0.10 g of water-absorbing resin particles 10a were uniformly disposed on the polyamide mesh 32 in the cylinder 31, the weight 33 was disposed on the water-absorbing resin particles 10a, and the cylinder 31 was installed such that the central portion thereof coincided with the opening of the conduit at the central portion of the measuring table 13. The decrease amount of the physiological saline in the burette tube 21 (that is, the amount of physiological saline absorbed by water-absorbing resin particles 10a) Wc (ml) was read at 60 minutes after the water-absorbing resin particles 10a started to absorb the physiological saline from the conduit 5, and the physiological saline absorption capacity under a load of 4.14 kPa of the water-absorbing resin particles 10a was calculated by the following equation.

Physiological saline absorption capacity under a load of 4.14 kPa (ml/g) = Wc (ml)/mass (g) of water-absorbing resin particles

<BET Specific Surface Area>

Water-absorbing resin particles to be measured were adjusted to have a particle size passed through a sieve with an aperture of 400 $\mu$m and retained on a sieve with an aperture of 300 $\mu$m, and used for measurement of the specific surface area. Next, 10 g of the classified sample was dispersed in 100 g of ethanol, washed with an ultrasonic washer (US-103, manufactured by SND Co., Ltd) for 5 minutes, and then filtered through a sieve with an aperture of 300 $\mu$m. The same washing operation was performed two more times to obtain a measurement sample washed three times in total. This sample was dried at 100°C for 16 hours under degassing conditions: heating and vacuum evacuation. Thereafter, an adsorption isotherm was measured at a temperature of 77 K by a method using krypton gas as an adsorption gas with a specific surface area analyzer (AUTOSORB-1, manufactured by Quantachrome Instruments), and a specific surface area was determined from a multipoint BET plot, and taken as the BET specific surface area of the water-absorbing resin particles.

[Preparation of Activated Carbon]

<Activated Carbon A>

Activated carbon (Osaka Gas Chemicals Co., Ltd., product name: LH2c 32/60SS) having a specific surface area of 1445 $m^2$/g, a median particle size of 456 $\mu$m, a residue on ignition of 0.3%, a loss on drying of 1.0%, an iodine adsorption amount of 1510 mg/g, a pH of 8.3, and a crushed shape was prepared. This was designated as activated carbon A.

<Activated Carbon B>

The activated carbon A was placed in an electric mixer (IJM-M800-W, manufactured by IRIS OHYAMA Inc.) and pulverized for 5 minutes. Next, the JIS standard sieves were combined in order of a sieve with an aperture of 212 $\mu$m, a sieve with an aperture of 106 $\mu$m, and a receptacle, and the pulverized activated carbon was put into the combined uppermost sieve and shaken for 10 minutes using a rotating and tapping shaker to perform classification. Thereafter, the activated carbon passed through a sieve with an aperture of 212 $\mu$m and retained on the sieve with an aperture of 106 $\mu$m was collected. The median particle size of the collected activated carbon was 159 $\mu$m. This was designated as activated carbon B.

<Activated Carbon C>

Activated carbon (Osaka Gas Chemicals Co., Ltd., product name: FP-3) having a specific surface area of 1619 $m^2$/g, a median particle size of 38 $\mu$m, a residue on ignition of 0.1%, a loss on drying of 0.8%, an iodine adsorption amount of 1560 mg/g, a pH of 7.1, and a crushed shape was prepared. This was designated as activated carbon C.

[Evaluation of Activated Carbon]

<Median Particle Size of Activated Carbon (Laser Diffraction)>

The median particle size (D50 (median diameter), volume-based) of the activated carbon used was measured with a laser diffraction particle size distribution analyzer (SALD 2300, manufactured by Shimadzu Corporation).

<Measurement of BET Specific Surface Area of Activated Carbon>

Using a pretreatment device (BELPREP VAC II, manufactured by MicrotracBEL Corp.), 0.1 g of the activated carbon to be measured was dried at 60°C for 24 hours under degassing conditions: heating and vacuum evacuation. Thereafter, an adsorption isotherm was measured at a temperature of 77 K by a method using nitrogen gas as an adsorption gas with a specific surface area analyzer (BELSORP MINI II, manufactured by MicrotracBEL Corp.), and a specific surface area was determined from a multipoint BET plot, and taken as the BET specific surface area of the activated carbon.

[Production of Water-Absorbing Resin Composition]

<Example 1>

Powder mixing of 0.1 parts of the activated carbon B as activated carbon with 100 parts by mass of the water-absorbing resin particles obtained in Production Example 1 was performed by rotation for 20 minutes under the conditions of a rotational speed of 50 rpm and a revolution speed of 50 rpm using a cross rotary mixer manufactured by Meiwa Kogyo Co., Ltd., to obtain a water-absorbing resin composition having an activated carbon content of 0.1 mass%.

<Example 2>

A water-absorbing resin composition having an activated carbon content of 0.05 mass% was obtained in the same manner as in Example 1 except that 0.05 parts by mass of the activated carbon A was used instead of the activated carbon B.

<Example 3>

A water-absorbing resin composition having an activated carbon content of 0.1 mass% was obtained in the same manner as in Example 2 except that the amount of the activated carbon A added was changed to 0.1 parts by mass.

<Example 4>

A water-absorbing resin composition having an activated carbon content of 1.0 mass% was obtained in the same manner as in Example 2 except that the amount of the activated carbon A added was changed to 1.01 parts by mass.

<Example 5>

A water-absorbing resin composition having an activated carbon content of 3.0 mass% was obtained in the same manner as in Example 2 except that the amount of the activated carbon A added was changed to 3.09 parts by mass.

<Comparative Example 1>

A water-absorbing resin composition having an activated carbon content of 0.1 mass% was obtained in the same manner as in Example 1 except that activated carbon C was used instead of the activated carbon B.

<Comparison 2>

A water-absorbing resin composition having an activated carbon content of 10 mass% was obtained in the same manner as in Example 2 except that the amount of the activated carbon A added was changed to 11.1 parts by mass.

<Comparative Example 3>

The water-absorbing resin particles obtained in Production Example 1 were used as Comparative Example 3 without addition of activated carbon.

[Evaluation of Water-Absorbing Resin Composition]

<Rate of Blocking due to Moisture Absorption>

The water-absorbing resin composition, 5.0 g, passed through a 20 mesh sieve was uniformly placed in an aluminum cylindrical dish having a diameter of 5 cm, which was allowed to stand for 3 hours in a thermo-hygrostat at a temperature of 30 ± 1°C and a relative humidity of 80 ± 5%. The total mass of the water-absorbing resin composition after standing for 3 hours a [g] was measured. Then, the water-absorbing resin composition was placed on a 12 mesh wire gauze (inner diameter: 20 cm), tapped 7 times by hand, and blocked by moisture absorption, and the mass of the water-absorbing resin composition retained on the 12 mesh wire gauze b [g] was measured, and the rate of blocking due to moisture absorption was determined from the following formula.

$$\text{Rate of blocking due to moisture absorption } [\%] = (b/a) \times 100$$

In addition, an improvement rate of blocking due to moisture absorption by adding activated carbon was determined by the following formula. Improvement rate of blocking due to moisture absorption [%] = [(rate of blocking due to moisture absorption of blank - rate of blocking due to moisture absorption of water-absorbing resin composition with activated carbon added)/rate of blocking due to moisture absorption of blank] × 100
wherein blank means that the same operation as the <Rate of Blocking due to Moisture Absorption> was performed using water-absorbing resin particles to which activated carbon was not added.

[Table 1]

EP 4 424 780 A1

| | Production Example | Water-absorbing resin particles | | | | | | Activated carbon | | | | Evaluation of water-absorbing resin composition | | |
|---|---|---|---|---|---|---|---|---|---|---|---|---|---|---|
| | | Physiological saline retention amount [g/g] | Physiological saline absorption amount under a load of 4.14 kPa [ml/g] | Water absorption speed [sec] | Median particle size [μm] | BET specific surface area [m²/g] | Bulk density [g/ml] | Activated carbon | Shape | Median particle size [μm] | Content in water-absorbing resin composition [%] | Rate of blocking due to moisture absorption [%] | Difference in blocking due to moisture absorption: blank - after addition of activated carbon | Improvement rate of blocking due to moisture absorption [%] |
| Example 1 | 1 | 39 | 22 | 35 | 348 | 0.032 | 0.70 | B | Crushed | 159 | 0.1 | 19 | 4 | 17 |
| Example 2 | 1 | 39 | 22 | 35 | 348 | 0.032 | 0.70 | A | Crushed | 456 | 0.05 | 19 | 4 | 17 |
| Example 3 | 1 | 39 | 22 | 35 | 348 | 0.032 | 0.70 | A | Crushed | 456 | 0.1 | 19 | 4 | 17 |
| Example 4 | 1 | 39 | 22 | 35 | 348 | 0.032 | 0.70 | A | Crushed | 456 | 1 | 11 | 12 | 52 |
| Example 5 | 1 | 39 | 22 | 35 | 348 | 0.032 | 0.70 | A | Crushed | 456 | 3 | 20 | 3 | 13 |
| Comparative Example 1 | 1 | 39 | 22 | 35 | 348 | 0.032 | 0.70 | C | Cm shed | 38 | 0.1 | 28 | -5 | -22 |
| Comparative Example 2 | 1 | 39 | 22 | 35 | 348 | 0.032 | 0.70 | A | Crushed | 456 | 10 | 28 | -5 | -22 |
| Comparative Example 3 | 1 | 39 | 22 | 35 | 348 | 0.032 | 0.70 | - | - | - | - | 23 | - | - |

16

DESCRIPTION OF REFERENCE SIGNS

1: Burette portion
3: Clamp
4: Measuring unit
5: Conduit
10a: Water-absorbing resin particles
11: Rack
13: Measuring table
13a: Through hole
15: Nylon mesh sheet
21: Burette tube
22: Cock
23: Rubber stopper
24: Cock
25: Air introduction tube
31: Cylinder
32: Polyamide mesh
33: Weight

**Claims**

1.  A water-absorbing resin composition comprising water-absorbing resin particles, and activated carbon having a median particle size of 100 $\mu$m or more and 600 $\mu$m or less,
    wherein a content of the activated carbon is 0.03 mass% or more and 5 mass% or less.

2.  The water-absorbing resin composition according to claim 1, wherein the activated carbon has a crushed shape.

3.  The water-absorbing resin composition according to claim 1 or 2, wherein the water-absorbing resin particles have a BET specific surface area of 0.01 m$^2$/g or more and 0.20 m$^2$/g or less.

4.  An absorber comprising the water-absorbing resin composition according to claim 1 or 2.

5.  An absorbent article comprising the absorber according to claim 4.

FIG. 1

# EP 4 424 780 A1

<table>
<tr><td colspan="2"><b>INTERNATIONAL SEARCH REPORT</b></td><td>International application No.<br><br><b>PCT/JP2022/040443</b></td></tr>
</table>

**A.     CLASSIFICATION OF SUBJECT MATTER**

*C08L 101/14*(2006.01)i; *A61F 13/15*(2006.01)i; *A61F 13/53*(2006.01)i; *A61L 15/18*(2006.01)i; *A61L 15/46*(2006.01)i;
*B01J 20/26*(2006.01)i; *B01J 20/28*(2006.01)i; *C08F 8/14*(2006.01)i; *C08K 3/04*(2006.01)i; *C08L 33/02*(2006.01)i
FI:    C08L101/14; C08K3/04; C08F8/14; B01J20/26 D; B01J20/28 Z; A61F13/15 141; A61F13/53 300; A61L15/46 200;
A61L15/18 200; C08L33/02

According to International Patent Classification (IPC) or to both national classification and IPC

**B.     FIELDS SEARCHED**

Minimum documentation searched (classification system followed by classification symbols)

C08K3/00-13/08; C08L1/00-101/14; A61F13/15-13/84; A61L9/00-9/22; A61L15/18; A61L15/46; B01J20/00-20/28;
B01J20/30-20/34; C08C19/00-19/44; C08F6/00-246/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C.     DOCUMENTS CONSIDERED TO BE RELEVANT**

| Category* | Citation of document, with indication, where appropriate, of the relevant passages | Relevant to claim No. |
|---|---|---|
| A | JP 2014-023766 A (LIVEDO CORPORATION) 06 February 2014 (2014-02-06)<br>entire text | 1-5 |
| A | JP 2000-093459 A (KAO CORP) 04 April 2000 (2000-04-04)<br>entire text | 1-5 |
| A | JP 61-101536 A (NIPPON SHOKUBAI KAGAKU KOGYO CO LTD) 20 May 1986<br>(1986-05-20)<br>entire text | 1-5 |
| A | JP 6-240151 A (NIPPON SHOKUBAI CO LTD) 30 August 1994 (1994-08-30)<br>entire text | 1-5 |
| A | JP 2016-502461 A (SCA HYGIENE PRODUCTS AB) 28 January 2016 (2016-01-28)<br>entire text | 1-5 |

☑ Further documents are listed in the continuation of Box C.          ☑ See patent family annex.

| | | |
|---|---|---|
| * | Special categories of cited documents: | "T"  later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X"  document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"  document member of the same patent family |

| Date of the actual completion of the international search | Date of mailing of the international search report |
|---|---|
| **20 December 2022** | **10 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
| --- |
| **PCT/JP2022/040443** |

### C.     DOCUMENTS CONSIDERED TO BE RELEVANT

| Category* | Citation of document, with indication, where appropriate, of the relevant passages | Relevant to claim No. |
| --- | --- | --- |
| A | JP 2005-533903 A (KIMBERLY-CLARK WORLDWIDE, INC.) 10 November 2005 (2005-11-10) entire text | 1-5 |
| A | JP 9-085082 A (BIO MEDEIKU KENKYUSHO KK) 31 March 1997 (1997-03-31) entire text | 1-5 |

Form PCT/ISA/210 (second sheet) (January 2015)

**INTERNATIONAL SEARCH REPORT**
Information on patent family members

International application No.

**PCT/JP2022/040443**

| Patent document cited in search report | | | Publication date (day/month/year) | Patent family member(s) | | | Publication date (day/month/year) |
|---|---|---|---|---|---|---|---|
| JP | 2014-023766 | A | 06 February 2014 | US | 2015/0246153 | A1 | |
| | | | | entire text | | | |
| | | | | WO | 2014/017100 | A1 | |
| | | | | EP | 2877137 | A1 | |
| | | | | CN | 104507437 | A | |
| JP | 2000-093459 | A | 04 April 2000 | US | 6663949 | B1 | |
| | | | | entire text | | | |
| | | | | WO | 2000/004938 | A1 | |
| | | | | EP | 1100552 | A1 | |
| | | | | CN | 1310634 | A | |
| JP | 61-101536 | A | 20 May 1986 | US | 4734478 | A | |
| | | | | entire text | | | |
| | | | | FR | 2566786 | A1 | |
| | | | | KR | 10-1990-0001551 | B1 | |
| JP | 6-240151 | A | 30 August 1994 | US | 5866678 | A | |
| | | | | entire text | | | |
| | | | | EP | 605215 | A1 | |
| JP | 2016-502461 | A | 28 January 2016 | US | 2015/0290052 | A1 | |
| | | | | entire text | | | |
| | | | | WO | 2014/074038 | A1 | |
| | | | | EP | 2916880 | A1 | |
| | | | | CN | 104780950 | A | |
| JP | 2005-533903 | A | 10 November 2005 | US | 2004/0024092 | A1 | |
| | | | | entire text | | | |
| | | | | WO | 2004/011044 | A1 | |
| | | | | EP | 1525008 | A1 | |
| | | | | CN | 1668344 | A | |
| | | | | CN | 101269236 | A | |
| | | | | KR | 10-2005-0025337 | A | |
| JP | 9-085082 | A | 31 March 1997 | (Family: none) | | | |

Form PCT/ISA/210 (patent family annex) (January 2015)

**REFERENCES CITED IN THE DESCRIPTION**

**Patent documents cited in the description**

- JP 2017185485 A